# EUROPÄISCHE PATENTSCHRIFT

(11) **EP 1 543 048 B1**
(45) Veröffentlichungstag und Bekanntmachung des Hinweises auf die Patenterteilung: **20.02.2008**
(21) Anmeldenummer: 03753410.4
(22) Anmeldetag: 12.09.2003
(51) Int. Cl.: C08F 26/02

(54) **WÄSSRIGE DISPERSIONEN VON WASSERLÖSLICHEN POLYMERISATEN VON N-VINYLCARBONSÄUREAMIDEN, VERFAHREN ZU IHRER HERSTELLUNG UND IHRE VERWENDUNG**
AQUEOUS DISPERSIONS OF WATER-SOLUBLE POLYMERS OF N-VINYLCARBOXAMIDES, METHOD FOR PRODUCTION AND USE THEREOF
DISPERSIONS AQUEUSES DE POLYMERISATS SOLUBLES DANS L'EAU DE N-VINYLCARBOXAMIDES, PROCEDE DE PRODUCTION ET D'UTILISATION ASSOCIE

(30) Priorität: 18.09.2002 DE 10243392
(43) Veröffentlichungstag der Anmeldung: 22.06.2005
(73) Patentinhaber: BASF Aktiengesellschaft, 67056 Ludwigshafen (DE)
(72) Erfinder: LEDUC, Marc, 67346 Speyer (DE)
(86) Internationale Anmeldenummer: PCT/EP2003/010153
(87) Internationale Veröffentlichungsnummer: WO 2004/029110

(56) Entgegenhaltungen:
- DE-A- 19 515 943
- DE-A- 19 606 899

## Beschreibung

Die Erfindung betrifft wässrige Dispersionen von wasserlöslichen Polymerisaten von N-Vinylcarbonsäureamiden, Verfahren zur Herstellung der Dispersionen in Gegenwart von Stabilisatoren und die Verwendung der Dispersionen als Entwässerungs-, Flockungs- und Retentionsmittel, als Nass- und Trockenfestigkeitsmittel und als Fixiermittel bei der Herstellung von Papier.

Aus der EP-B-0 814 099 ist ein Verfahren zur Herstellung einer wässrigen Dispersion eines wasserlöslichen kationischen Polymeren bekannt, wobei man eine wässrige, Neutralsalze enthaltende Dispersion eises Vinylformamideinheiten enthaltenden Polymeren mit einer Säure in wässrigem Medium unter Zusatz von Nitraten hydrolysiert. Die so hergestellten wässrigen Dispersionen von Vinylamineinheiten enthaltenden Polymerisaten haben einen hohen Neutralsalzgehalt.

Aus der WO-A-97/30094 ist ein Verfahren zur Herstellung von Dispersionen wasserlöslicher kationischer Vinylpolymerer bekannt. Die Polymerisation der wasserlöslichen Monomeren erfolgt in wässrigen Salzlösungen in Gegenwart von Stabilisatoren, die aus einem wasserlöslichen Pfropfcopolymer bestehen, das als Pfropfgrundlage Polyethylenoxid und als Seitenketten aufgepfropfte kationische Vinylmonomere enthält.

Aus der WO-A-98/54234 sind wässrige Dispersionen von wasserlöslichen N-Vinylcarbonsäureamiden bekannt. Die Dispersionen werden durch Polymerisieren von N-Vinylcarbonsäureamiden in wässrigem Medium in Gegenwart wasserlöslicher Salze und wasserlöslicher polymerer Stabilisatoren hergestellt. Als polymere Stabilisatoren werden beispielsweise Polyvinylalkohol und partiell hydrolysierte Polyvinylacetate eingesetzt.

Gegenstand der WO-A-00/27893 sind wässrige Dispersionen von wasserlöslichen Polymerisaten von N-Vinylcarbonsäureamiden, Verfahren zu ihrer Herstellung und die Verwendung als Entwässerungs-, Flockungs- und Retentionsmittel sowie als Nass- und Trockenfestigkeitsmittel und als Fixiermittel bei der Herstellung von Papier. Zur Stabilisierung der Dispersionen verwendet man polymere Dispergiermittel, z.B. Polyethylenglykol, Polypropylenglykol, Polyvinylacetat, Polyvinylalkohol, Polyvinylpyridin, Polyvinylimidazol und Polydiallyldimethylammoniumchlorid.

Aus der WO-A-96/34903 sind Pfropfpolymere bekannt, die durch Polymerisieren von N-Vinylcarbonsäureamiden wie N-Vinylformamid und gegebenenfalls anderen damit copolymerisierbaren Monomeren auf Polyalkylenoxiden und/oder Polytetrahydrofuranen und anschließende zumindest teilweise Abspaltung von beispielsweise Formylgruppen aus den aufgepfropften Vinylformamideinheiten unter Bildung von Vinylamineinheiten erhältlich sind. Die Pfropfpolymerisate werden beispielsweise als Trocken- und Naßverfestigungsmittel, als Fixiermittel für Störstoffe und Füllstoffe, als Retentions- und Entwässerungsmittel bei der Herstellung von Papier sowie als Promoter bei der Dikenleimung von Papier und als Hilfsmittel bei der Herstellung von Tissuepapieren verwendet.

Aus der älteren, nicht vorveröffentlichten DE-Anmeldung WO 03/046024 sind wässrige Dispersionen von wasserlöslichen Polymerisaten von N-Vinylcarbonsäureamiden bekannt. Diese Dispersionen enthalten als Stabilisator wasserlösliche, vernetzte Pfropfpolymerisate, die durch radikalisch initiierte Polymerisation von Verbindungen mit mindestens zwei ethylenisch ungesättigten Doppelbindungen in Gegenwart von Polyalkylenglykolen erhältlich sind. Sie werden beispielsweise bei der Papierherstellung als Entwässerungs-, Flockungs- und Retentionsmittel, als Naß- und Trockenfestigkeitsmittel und als Fixiermittel verwendet.

Der Erfindung liegt die Aufgabe zugrunde, weitere Dispersionen von wasserlöslichen Polymerisaten von N-Vinylcarbonsäureamiden zur Verfügung zu stellen.

Die Aufgabe wird erfindungsgemäß gelöst mit wässrigen Dispersionen von wasserlöslichen Polymerisaten von N-Vinylcarbonsäureamiden, wenn die Dispersionen als Stabilisator wasserlösliche Pfropfpolymere enthalten, die durch radikalisch initiierte Polymerisation von Monomeren oder Monomergemischen aus
- 10 bis 100 Gew.-% N-Vinylcarbonsäureamiden der Formel

   CH₂=CH-NR²-CO-R¹ (I),

   in der R¹, R² = H und /oder C₁- bis C₆-Alkyl bedeuten,und
- 0 bis 90 Gew.-% anderen, mit den Monomeren der Formel I copolymerisierbaren Monomeren in Gegenwart von Polymerisaten, die mindestens 3 Einheiten eines C₂- bis C₄-Alkylenoxids enthalten und/oder von Polytetrahydrofuranen, wobei das Molekulargewich der Polyalkylenglykole im Bereich kleiner 500 000 liegt (nach Zahlenmittel)
erhältlich sind.

Die Aufgabe wird außerdem mit solchen wässrigen Dispersionen gelöst, die als Stabilisator hydrolysierte Pfropfpolymere enthalten, die durch Abspaltung der Gruppe -CO-R¹ aus den aufgepfropften N-Vinylcarbonsäureamideinheiten des Stabilisators unter Bildung von Vinylamineinheiten erhältlich sind.

Gegenstand der Erfindung ist auch ein Verfahren zur Herstellung von wässrigen Dispersionen von wasserlöslichen N-Vinylcarbonsäureamiden durch radikalische Polymerisation von N-Vinylcarbonsäureamiden in wässrigem Medium in Gegenwart von Stabilisatoren, wenn man als Stabilisator und/oder Fällungsmittel wasserlösliche Pfropfpolymere einsetzt, die durch radikalisch initiierte Polymerisation von Monomeren oder Monomergemischen aus
- 10 bis 100 Gew.-% N-Vinylcarbonsäureamiden der Formel

   CH₂=CH-NR²-CO-R¹ (I),

   in der R¹, R² = H und /oder C₁- bis C₆-Alkyl bedeuten,und
- 0 bis 90 Gew.-% anderen, mit den Monomeren der Formel I copolymerisierbaren Monomeren in Gegenwart von Polymerisaten, die mindestens 3 Einheiten eines C₂- bis C₄-Alkylenoxids enthalten und/oder von Polytetrahydrofuranen, wobei das Molekulargewich der Polyalkylenglykole im Bereich kleiner 500 000 liegt (nach Zahlenmittel)
erhältlich sind.

Um die wasserlöslichen Polymerisate von Poly-N-Vinylcarbonsäureamiden aus der wässrigen Lösung während des Polymerisationsvorgangs auszufällen, führt man die Polymerisation vorzugsweise in Gegenwart von wasserlöslichen polymeren Verbindungen aus, die die entstehenden Poly-N-Vinylcarbonsäureamide aus der wässrigen Lösung ausfällen, z.B. in Gegenwart von Polyalkylenglykolen, Polyvinylalkoholen, Polyvinylacetet, Polyvinylpyridin, Polyvinylimidazol, Carboxylmethylcellulose und/oder Polytetrahydrofuran. Bevorzugt eingesetztes Fällungsmittel ist Polyethylenglykol. Die Fällungsmittel werden, sofern man sie bei der Polymerisation überhaupt einsetzt, in Mengen von 1 bis 50, insbesondere 5 bis 30 Gew.-%, bezogen auf die gesamte Dispersion, eingesetzt. Solche Fällungsmittel bzw. Dispergiermittel werden in der WO-A-00/27893, Seite 4, Zeilen 38-44 und Seite 5, Zeilen 1 bis 9 beschrieben. 100 Gew.-Teile der Dispersionen enthalten beispielsweise (a) 5 bis 60 Gew.-Teile eines wasserlöslichen Polymerisats eines N-Vinylcarbonsäureamids und (b) 0,1 bis 30 Gew.-Teile mindestens eines Pfropfpolymerisats von N-Vinylcarbonsäureamiden auf Polyalkylenglykolen und/oder Polytetrahydrofuranen erhältlich sind. Die wasserlöslichen Polymerisate von N-Vinylcarbonsäureamiden basieren vorzugsweise auf Homo- und Copolymerisaten, die Einheiten der Formel 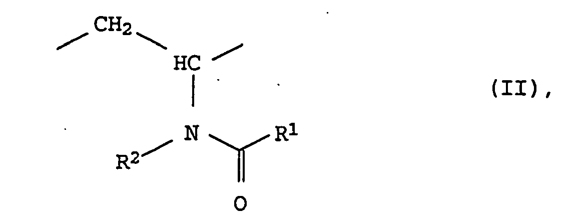 einpolymerisiert enthalten, in der R¹, R² = H und/oder C₁- bis C₆-Alkyl bedeuten. Die Polymeren werden durch Homo- oder Copolymerisation von z.B. N-Vinylformamid, N-Vinylacetamid, N-Vinyl-N-methylacetamid oder N-Vinyl-N-methylformamid hergestellt. Von den N-Vinylcarbonsäureamiden-wird N-Vinylformamid bevorzugt eingesetzt.

Die wasserlöslichen N-Vinylcarbonsäureamid-Einheiten enthaltenden Polymerisate können gegebenenfalls 1 bis 80, vorzugsweise 5 bis 30 Gew.-% an weiteren Monomeren copolymerisiert enthalten. Solche Monomere sind beispielsweise monoethylenisch ungesättigte Carbonsäuren mit 3 bis 8 C-Atomen wie Acrylsäure, Methacrylsäure, Dimethacrylsäure, Ethacrylsäure, Maleinsäure, Citraconsäure, Methylenmalonsäure, Allylessigsäure, Vinylessigsäure, Crotonsäure, Fumarsäure, Mesaconsäure und Itaconsäure. Aus dieser Gruppe von Monomeren verwendet man vorzugsweise Acrylsäure, Methacrylsäure, Maleinsäure oder Mischungen der genannten Carbonsäuren. Die monoethylenisch ungesättigten Carbonsäuren werden entweder in Form der freien Säuren oder in Form ihrer Alkalimetall-, Erdalkalimetall- oder Ammoniumsalze bei der Copolymerisation eingesetzt. Zur Neutralisation der freien Carbonsäuren verwendet man vorzugsweise Natronlauge, Kalilauge, Soda, Pottasche, Natriumhydrogencarbonat, Magnesiumoxid, Calciumhydroxid, Calciumoxid, gasförmiges oder wässriges Ammoniak, Triethylamin, Ethanolamin, Diethanolamin, Triethanolamin, Morpholin, Diethylentriamin oder Tetraethylenpentamin.

Weitere geeignete Comonomere sind beispielsweise die Ester, Amide und Nitrile der obenangegebenen Carbonsäuren, z.B. Acrylsäuremethylester, Acrylsäureethylester, Methacrylsäuremethylester, Methacrylsäurethylester, Hydroxyethylacrylat, Hydroxypropylacrylat, Hydroxybutylacrylat, Hydroxyethylmethacrylat, Hydroxypropylmethacrylat, Hydroxyisobutylacrylat, Hydroxyisobutylmethacrylat, Maleinsäuremonomethylester, Maleinsäuredimethylester, Maleinsäuremonoethyslester, Maleinsäurediethylester, 2-Ethylhexylacrylat, 2-Ethylhexylmethacrylat, Acrylamid, Methacrylamid, N-Dimethylacrylamid, N-tert.-Butylacrylamid, Acrylnitril, Methacrylnitril, Dimethylaminoethylacrylat, Diethylaminoethylacrylat, Diethylaminoethylmethacrylat sowie die Salze der zuletzt genannten basischen Monomeren mit Carbonsäuren oder Mineralsäuren sowie die quaternierten Produkte der basischen (Meth)acrylate.

Außerdem eignen sich als andere copolymerisierbare Monomere Acrylamidoglykolsäure, Vinylsulfonsäure, Allylsulfonsäure, Methallylsulfonsäure, Styrolsulfonsäure, Acrylsäure-(3-sulfopropyl)ester, Methacrylsäure-(3-sulfopropyl)ester und Acrylamidomethylpropansulfonsäure sowie Phosphonsäuregruppen enthaltende Monomere wie Vinylphosphonsäure, Allylphosphonsäure und Acrylamidomethanpropanphosphonsäure. Die Säuregruppen enthaltenden Monomeren können in Form der freien Säuregruppen sowie in partiell oder in vollständig mit Basen neutralisierter Form bei der Polymerisation eingesetzt werden.

Weitere geeignete copolymerisierbare Verbindungen sind N-Vinylpyrrolidon, N-Vinylcaprolactam, N-Vinylimidazol, N-Vinyl-2-methylimidazol, N-Vinyl-4-methylimidazol, Diallylammoniumchlorid, Vinylacetat, Vinylpropionat und Styrol. Es ist selbstverständlich auch möglich, Mischungen der genannten Monomeren einzusetzen. Sofern die genannten Monomeren bei alleiniger Polymerisation keine wasserlöslichen Polymerisate ergeben, enthalten die N-Vinylcarbonsäureamid-Einheiten enthaltenden Polymerisate diese Comonomeren nur in solchen Mengen einpolymerisiert, daß die Copolymerisate noch wasserlöslich sind.

Im Gegensatz zu Wasser-in-Ö1-Polymeremulsionen sind für die erfindungsgemäßen wässrigen Dispersionen keine organischen Lösemittel erforderlich. Wie aus dem eingangs angegebenen Stand der Technik hervorgeht, sind konzentrierte Lösungen anorganischer Salze ein übliches Mittel, um wässrige Dispersionen von wasserlöslichen Polymerisaten herzustellen. Dadurch enthalten die bekannten Dispersionen eine sehr hohe Salzfracht. Der Vorteil der erfindungsgemäßen wässrigen Dispersionen von wasserlöslichen Polymerisaten liegt darin, dass sie gegenüber den bekannten wässrigen Dispersionen praktisch salzfrei hergestellt werden können. Die wässrigen Dispersionen wasserlöslicher Polymerisate von N-Vinylformamid, N-Vinyl-N-methylformamid, N-Vinyl-N-methylacetamid und/oder N-Vinylacetamid besitzen vorzugsweise einen hohen Polymerisatgehalt und enthalten vorzugsweise Polymerisate mit hohen Molmassen bei gleichzeitig geringer Viskosität. Die Molmassen M_{w} der N-Vinylcarbonsäureamid-Einheiten enthaltenden Polymerisate betragen beispielsweise 5 · 10⁴ bis 1 · 10⁷, vorzugsweise 2 · 10⁵ bis 1 . 106.

Die als Stabilisator und/oder Fällungsmittel in den wässrigen Dispersionen von wasserlöslichen Polymeren von N-Vinylcarbonsäureamiden enthaltenen Pfropfpolymerisate sind aus der zum Stand der Technik genannten WO-A-96/34903 bekannt. Die erfindungsgemäßen wässrigen Dispersionen können sowohl hydrolysierte als auch nicht hydrolysierte Pfropfpolymerisate von N-Vinylcarbonsäureamiden auf Polyalkylenglykolen und/oder Polytetrahydrofuranen enthalten. Die Stabilisatoren sind erhältlich durch radikalisch initiierte Polymerisation von Monomeren oder Monomergemischen aus
- 10 bis 100 Gew.-% N-Vinylcarbonsäureamiden der Formel

   CH₂=CH-NR²-CO-R¹ (I),

   in der _{R}¹, R² = H und /oder C₁- bis C₆-Alkyl bedeuten, und
- 0 bis 90 Gew.-% anderen, mit den Monomeren der Formel I copolymerisierbaren Monomeren in Gegenwart von Polymerisaten, die mindestens 3 Einheiten eines C₂- bis C₄-Alkylenoxids enthalten und/oder von Polytetrahydrofuranen.

Bezogen auf 100 Gewichtsteile des als Pfropfgrundlage eingesetzten Polymerisats verwendet man beispielsweise 5 bis 500, vorzugsweise 10 bis 400 Gewichtsteile mindestens eines Monomeren. Man kann jedoch auch aus den so erhältlichen Pfropfpolymerisaten die Gruppe -CO - R¹ durch Hydrolyse abspalten und die dabei entstehenden Vinylamineinheiten enthaltenden Produkte als Stabilisator verwenden. Die in den Pfropfpolymeren enthaltenen N-Vinylcarbonsäureamideinheiten können zu 1 bis 100, vorzugsweise 5 bis 95 Mol-% hydrolysiert sein. Die Herstellung der hydrolysierten Pfropfpolymerisate wird in der WO-A-96/34903 ausführlich beschrieben, vgl. insbesondere die Beispiele.

Für die Herstellung des Stabilisators eignet sich insbesondere N-Vinylformamid als Monomer. Falls man Copolymerisate verwendet, so setzt man beispielsweise Copolymerisate aus N-Vinylformamid und mindestens einem der folgenden Comonomere ein:
- N-Vinylimidazol-Derivate der allgemeinen Formel (III) 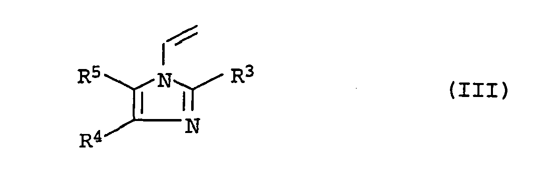 worin R³, R⁴ und R⁵ unabhängig voneinander für Wasserstoff, C₁-C₄-Alkyl- oder Phenyl stehen, bevorzugt 2-Methyl-N-vinylimidazol oder N-Vinylimidazol
- N,N-Diallylamine der allgemeinen Formel (IV), 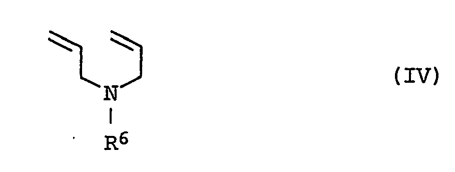
worin R⁶ für einen C₁-C₂₄-Alkylrest steht, bevorzugt N,N-Diallyl-N-methylamin
N,N-Diallylaminoalkylderivate der Acryl- oder Methacrylsäure der allgemeinen Formel (V) 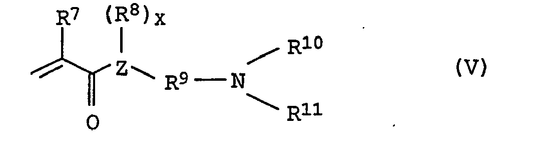 worin R⁷ und R⁸ unabhängig voneinander für Wasserstoff oder Methyl stehen, Z ein Stickstoffatom mit x = 0 bedeutet, R⁹ für einen linearen oder verzweigten C₁-C₂₄-Alkylenrest steht, und R¹⁰ und R¹¹ unabhängig voneinander für einen C₁-C₂₄-Alkylenrest stehen.

Beispiele für Verbindungen der allgemeinen Formel (III) sind folgender Tabelle 1 zu entnehmen:

**Tabelle 1**

| R³ | R⁴ | R⁵ |
|---|---|---|
| H | H | H |
| Me | H | H |
| H | Me | H |
| H | H | Me |
| Me | Me | H |
| H | Me | Me |
| Me | H | Me |
| Ph | H | H |
| H | Ph | H |
| H | H | Ph |
| Ph | Me | H |
| Ph | H | Me |
| Me | Ph | H |
| H | Ph | Me |
| H | Me | Ph |
| Me | H | Ph |

| | | |
|---|---|---|
| Me = Methyl Ph = Phenyl | | |

Weitere brauchbare Comonomere der Formel (III) sind die Ethyl-, Propyl- oder Butyl-Analoga der in Tabelle 1 aufgelisteten Methylsubstituierten 1-Vinylimidazole.

Beispiele für Verbindungen der allgemeinen Formel (IV) sind Diallylamine, worin R⁶ für Methyl, Ethyl, iso- oder n-Propyl, iso-, n- oder tert.-Butyl, Pentyl, Hexyl, Heptyl, Octyl, Nonyl oder Decyl steht. Beispiele für längerkettige Reste R⁶ sind Undecyl, Dodecyl, Tridecyl, Pentadecyl, Octadecyl und Icosayl.

Geeignete Comonomere der allgemeinen Formel (V) sind beispielsweise N,N-Dimethylaminomethyl(meth)acrylat, N,N-Diethylamino-methyl(meth)acrylat, N,N-Dimethylaminoethyl(meth)acrylat, N,N-Diethylaminoethyl(meth)acrylat, N,N-Dimethylaminobutyl-(meth)acrylat, N,N-Diethylaminobutyl(meth)acrylat, N,N-Dimethyl-aminohexyl(meth)acrylat, N,N-Dimethylaminooctyl(meth)acrylat, N,N-Dimethylaminododecyl(meth)acrylat, N-[3-(dimethylamino)-propyl]acrylamid, N-[3-(dimethylamino)butyl]methacrylamid, N-[8-(dimethylamino)-octyl]methacrylamid, N-[12-(dimethylamino)-dodecyl]methacrylamid, N-[3-(diethylamino)propyl]methacrylamid oder N-[3-(diethylamino)propyl]acrylamid, oder deren Gemische.

Bevorzugte Comonomere sind 3-Methyl-1-vinylimidazoliumchlorid und -methosulfat, Dimethyldiallylammoniumchlorid sowie N,N-Dimethylaminoethylmethacrylat und N-[3-(dimethylamino)propyl]methacrylamid, die wahlweise durch Methylchlorid, Dimethylsulfat oder Diethylsulfat quaternisiert wurden.

Besonders bevorzugte Comonomere sind 3-Methyl-1-vinylimidazolium-chlorid und -methosulfat und Dimethyldiallylammoniumchlorid, ganz besonders bevorzugt sind 3-Methyl-1-vinylimidazoliumchlorid und -methosulfat.

Zur Quaternisierung der Verbindungen der allgemeinen Formeln (III) bis (V) eignen sich beispielsweise Alkylhalogenide mit 1 bis 24 C-Atomen in der Alkylgruppe, z.B. Methylchlorid, Methylbromid, Methyliodid, Ethylchlorid, Ethylbromid, Propylchlorid, Hexylchlorid, Dodecylchlorid, Laurylchlorid und Benzylhalogenide, insbesondere Benzylchlorid und Benzylbromid. Weitere geeignete Quaternierungsmittel sind Dialkylsulfate, insbesondere Dimethylsulfat oder Diethylsulfat. Die Quaternierung der basischen Monomere der allgemeinen Formeln (III) bis (V) kann auch mit Alkylenoxiden wie Ethylenoxid oder Propylenoxid in Gegenwart von Säuren durchgeführt werden.

Die Quaternisierung des Comonomeren oder eines Polymeren mit einem der genannten Quaternisierungsmittel kann nach allgemein bekannten Methoden erfolgen.

Bevorzugte Quaternierungsmittel sind: Methylchlorid, Dimethylsulfat oder Diethylsulfat.

Als weitere Comonomere werden solche Verbindungen bevorzugt, die sich bei einer Temperatur von 25°C in Wasser zu mehr als 5 Gew.-% lösen.

Geeignete wasserlösliche Comonomere sind N-Vinyllactame, z.B. N-Vinylpiperidon, N-Vinylpyrrolidon und N-Vinylcaprolactam, N-Vinylacetamid, N-Methyl-N-vinylacetamid, Vinylformiat, Vinylacetat, Acrylsäuremethylester, Acrylsäureethylester, Methacrylsäuremethylester, Acrylsäure oder Methacrylsäure in Form der freien Säuren oder in Form ihrer Alkalimetall-, Erdalkalimetall- oder Ammoniumsalze, Acrylamid, Methacrylamid, N,N-Dimethylacrylamid, N-Methylolmethacrylamid, N-Vinyloxazolidon, N-Vinyltriazol, Hydroxyalkyl(meth)acrylate, z.B. Hydroxyethyl(meth)acrylat und Hydroxypropyl(meth)acrylate, oder Alkylethylengly-kol(meth)acrylate mit 1 bis 50 Ethylenglykoleinheiten im Molekül.

Besonders bevorzugt werden als solche Comonomere N-Vinyllactame eingesetzt. Ganz besonders bevorzugt ist N-Vinylpyrrolidon.

Als Polymere, die mindestens 3 Einheiten eines Alkylenoxids enthalten und/oder Polytetrahydrofurane kommen sämtliche Verbindungen in Betracht, die in der WO-A-96/34903, Seiten 8 bis 10 genannt sind. Solche Verbindungen haben beispielsweise ein mittleres Molekulargewicht (Zahlenmittel) von mindestens 300. Sie können beispielsweise mit Hilfe der folgenden Formel beschrieben werden: 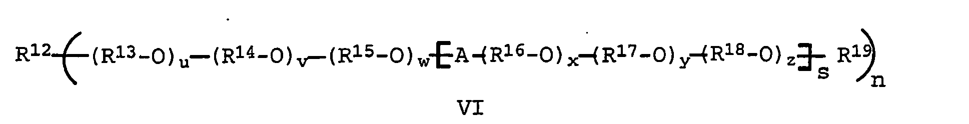 in der die Variablen unabhängig voneinander folgende Bedeutung haben:
- R¹²: Wasserstoff, NH₂, C₁-C₂₄-Alkyl, R²⁰-C(=O)-, R²⁰-NH-C(=O)-, Polyalkoholrest;
- R¹⁹: Wasserstoff, NH₂, C₁-C₂₄-Alkyl, R²⁰-C(=O)-, R²⁰-NH-C(=O)-;
- R¹³: bis R¹⁸ -(CH₂)₂-, -(CH₂)₃-, -(CH₂)₄-, -CH₂-CH(CH₃)-, -CH₂-CH(CH₂-CH₃)-, -CH₂-CHOR²¹-CH₂-;
- R²⁰: C₁-C₂₄-Alkyl;
- R²¹: Wasserstoff, C₁-C₂₄-Alkyl, R²⁰-C(=O)-;
- A: -C(=O)-O-, -C(=O)-B-C(=O)-O-, -C(=O)-NH-B-NH-C(=O)-O-;
- B: -(CH₂)ₜ-, Arylen, gegebenenfalls substituiert;
- n: 1 bis 8;
- s: 0 bis 500;
- t: 1 bis 12;
- u: 1 bis 5000;
- v: 0 bis 5000;
- w: 0 bis 5000;
- x: 1 bis 5000;
- y: 0 bis 5000;
- z: 0 bis 5000.

Solche Verbindungen sind aus dem Stand der Technik bekannt, vgl. WO-A-00/18375. Als Pfropfgrundlage werden generell Polyalkylenglykole der allgemeinen Formel VI, ausgewählt aus der Gruppe bestehend aus Polyalkylenoxiden auf Basis von Ethylenoxid, Propylenoxid und Butylenoxid sowie Polytetrahydrofuran verwendet. Je nach Art der Monomerbausteine ergeben sich Polymere mit folgenden Struktureinheiten:

-(CH₂)₂-O-, -(CH₂)₃-O-, -(CH₂)₄-O-, -CH₂-CH(CH₃)-O-,

-CH₂-CH(CH₂-CH₃)-O-, -CH₂-CHOR²¹-CH₂-O-;

Dabei kann es sich sowohl um Homopolymere als auch um Copolymere handeln, wobei die Copolymere statistisch verteilt sein können oder als sogenannte Blockpolymere vorliegen.

Die endständigen primären Hydroxylgruppen der, auf Basis von Alkylenoxiden hergestellten Polyalkylenglykole können sowohl frei vorliegen als auch ein- oder beidseitig mit Alkoholen einer Kettenlänge C₁-C₂₄ bzw. mit Carbonsäuren einer Kettenlänge C₁-C₂₄ verethert bzw. verestert sein. Sie können jedoch auch durch reduktive Aminierung mit Wasserstoff-Ammoniak-Gemischen unter Druck gegen primäre Aminogruppen ausgetauscht oder durch Cyanethylierung mit Acrylinitril und Hydrieren in Aminopropylendgruppen umgewandelt sein.

Als Alkylreste für R¹² und R¹⁹ bis R²¹ seien verzweigte oder unverzweigte C₁-C₂₄-Alkylketten, bevorzugt Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, 2-Ethylhexyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl oder n-Eicosyl genannt.

Als bevorzugte Vertreter der oben genannten Alkylreste seien verzweigte oder unverzweigte C₁-C₁₂-, besonders bevorzugt C₁-C₆-Alkylketten genannt.

Das Molekulargewicht der Polyalkylenglykole liegt im Bereich kleiner 500000 (nach Zahlenmittel), bevorzugt im Bereich von 300 bis 100000, besonders bevorzugt im Bereich von 500 bis 20000, ganz besonders bevorzugt im Bereich von 800 bis 15000 (jeweils Zahlenmittel des Molekulargewichts).

Vorteilhafterweise verwendet man Homopolymerisate des Ethylenoxids oder Copolymerisate, mit einem Anteil an Ethylenoxideinheiten von 40 bis 99 Gew.-%. Für die bevorzugt einzusetzenden Ethylenoxidpolymerisate beträgt somit der Anteil an einpolymerisiertem Ethylenoxid 40 bis 100 Mol-%. Als Comonomer für diese Copolymerisate kommen Propylenoxid, Butylenoxid und/oder Isobutylenoxid in Betracht. Geeignet sind beispielsweise Copolymerisate aus Ethylenoxid und Propylenoxid, Copolymerisate aus Ethylenoxid und Butylenoxid sowie Copolymerisate aus Ethylenoxid, Propylenoxid und mindestens einem Butylenoxid. Der Ethylenoxidanteil der Copolymerisate beträgt vorzugsweise 40 bis 99 Mol.-%, der Propylenoxidanteil 1 bis 60 Mol.-% und der Anteil an Butylenoxid in den Copolymerisaten 1 bis 30 Mol.-%. Neben geradkettigen können auch verzweigte Homo- oder Copolymerisate als Pfropfgrundlage verwendet werden.

Auch verzweigte Polymerisate sind ebenfalls als Pfropfgrundlage geeignet. Sie können hergestellt werden, indem man beispielsweise an niedrigmolekularen Polyalkoholresten (= R¹ in der allgemeinen Formel I, wie z.B. Pentaerythrit, Glycerin oder an Zuckern bzw. Zuckeralkoholen wie Saccharose, D-Sorbit und D-Mannit) Ethylenoxid und gegebenenfalls noch Propylenoxid und/oder Butylenoxide anlagert.

Dabei können Polymerisate gebildet werden, bei denen mindestens eine, bevorzugt eine bis acht, besonders bevorzugt eine bis fünf der in den Polyalkoholen vorhandenen Hydroxylgruppen in Form einer Etherbindung mit dem folgenden Polyetherrest P, gemäß Formel VI 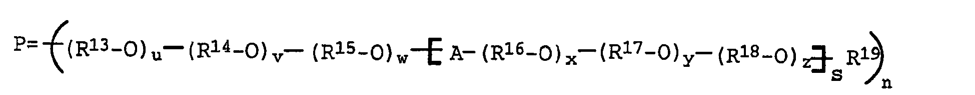 n = 1 bis 8 verknüpft sein können.

Die Alkylenoxid-Einheiten können im Polymerisat statistisch verteilt sein oder in Form von Blöcken vorliegen.

Es ist aber auch möglich, Polyester von Polyalkylenoxiden und aliphatischen C₁-C₁₂-, bevorzugt C₁-C₆-Dicarbonsäuren oder aromatischen Dicarbonsäuren, z.B. Oxalsäure, Bernsteinsäure, Adipinsäure oder Terephthalsäure mit Molmassen von 1500 bis 25000, beschrieben in EP-A-0 743 962, als Pfropfgrundlage zu verwenden.

Es ist weiterhin möglich, durch Phosgenierung hergestellte Polycarbonate von Polyalkylenoxiden oder auch Polyurethane von Polyalkylenoxiden und aliphatischen C₁-C₁₂-, bevorzugt C₁-C₆-Diisocyanaten oder aromatischen Diisocyanaten, z.B. Hexamethylendiisocyanat oder Phenylendiisocyanat als Pfropfgrundlage zu verwenden.

Die o.g. Polyester, Polycarbonate oder Polyurethane können bis zu 500, bevorzugt bis zu 100 Alkylenoxideinheiten enthalten, wobei die Alkylenoxideinheiten sowohl aus Homopolymeren als auch aus Copolymeren unterschiedlicher Alkylenoxide bestehen können.

Die wässrigen Dispersionen enthalten z.B. auf 100 Gew.-Teile Wasser 1 bis 50, vorzugsweise 5 bis 40 Gew.-Teile mindestens eines Stabilisators. Um wässrige Dispersionen von N-Vinylcarbonsäureamiden herzustellen, polymerisiert man in einer bevorzugten Auführungsform
- 5 bis 80 Gew.-Teile N-Vinylformamid und/oder N-Vinylacetamid gegebenenfalls zusammen mit anderen monoethylenisch ungesättigten Monomeren, die damit wasserlösliche Polymerisate bilden, und
- 1 bis 50 Gew.-Teile mindestens eines polymeren Stabilisators,
in 100 Gew.-Teilen Wasser bei Temperaturen von 30 bis 95°C in Gegenwart von 0,001 bis 5,0 Gew.-%, bezogen auf die eingesetzten Monomeren, mindestens eines Initiators.

In der besonders bevorzugten Ausführungsform des Verfahrens polymerisiert man
- 10 bis 50 Gew.-Teile N-Vinylformamid gegebenenfalls zusammen mit anderen monoethylenisch ungesättigten Monomeren, die damit wasserlösliche Polymerisate bilden, und
- 5 bis 40 Gew.-Teile mindestens eines polymeren Stabilisators
in 100 Gew.-Teilen Wasser bei Temperaturen von 40 bis 70°C mit 0,5 bis 2,0 Gew.-%, bezogen auf die bei der Polymerisation eingesetzten Monomeren, an Azoverbindungen, die unter den Polymerisationsbedingungen in Radikale zerfallen.

Die Monomeren werden erfindungsgemäß radikalisch polymerisiert, d.h. man verwendet Polymerisationsinitiatoren, die unter den Polymerisationsbedingungen Radikale bilden. Geeignete Verbindungen dieser Art sind beispielsweise Wasserstoffperoxid, Peroxide, Hydroperoxide, Redoxkatalysatoren und nicht oxidierend wirkende Initiatoren wie Azoverbindungen, die unter den Polymerisationsbedingungen in Radikale zerfallen. Geeignete Azoverbindungen sind beispielsweise 2,2'-Azo-bis(2-amidionopropan)dihydrochlorid, 2,2'-Azo-bis(N,N'-dimethylenisobutyramidin)dihydrochlorid, 2,2'-Azo-bis(2,4-dimethylvaleronitril), 2,2'-Azo-bis[2-methyl-N-(2-hydroxyethyl)-propionamid] oder 2,2'-Azo-bis-isobutyronitril. Es ist selbstverständlich auch möglich, Mischungen verschiedener Initiatoren einzusetzen. Diese Initiatoren können auch bei der Herstellung der Stabilisatoren verwendet werden. Bei der Herstellung der Dispersionen liegt der pH-Wert beispielsweise in dem Bereich von 5 bis 8.

Falls Dispersionen von Polymerisaten mit niedrigen Molekulargewichten gewünscht werden, kann man beispielsweise die Initiatormengen, die bei der Polymerisation üblicherweise eingesetzt werden, erhöhen, so daß man auch Initiatormengen einsetzen kann, die außerhalb des oben angegebenen Bereichs für die Initiatormengen liegen. Wässrige Dispersionen von niedrigmolekularen Homo- und Copolymerisaten der in Betracht kommenden Vinylcarbonsäureamide können auch dadurch erhalten werden, daß man die Polymerisation in Gegenwart von Polymerisationsreglern durchführt und gegebenenfalls gleichzeitig höhere Menge als üblicherweise erforderlich an Initiatoren einsetzt. Geeignete Polymerisationsregler sind beispielsweise Schwefel in gebundener Form enthaltende Verbindungen wie Dodecylmercaptan, Thioglykolsäure, Thioessigsäure und Mercaptoalkohole wie Mercaptoethanol, Mercaptopropanole und Mercaptobutanole. Daneben kann man als Polymerisationsregler auch Ameisensäure, Isopropanol und Hydrazin in Form von Salzen mit starken Säuren verwenden.

Die Molekulargewichte der in der dispergierten Form vorliegenden Polymerisate können auch mit Hilfe der K-Werte nach Fikentscher charakterisiert werden. Die K-Werte betragen bis zu 300 und liegen vorzugsweise in dem Bereich von 130 bis 180. Aus Lichtstreuexperimenten folgt, daß ein K-Wert von 250 einem mittleren Molekulargewicht der Polymerisate von etwa 7 000 000 Dalton entspricht.

Vinylcarbonsäureamideinheiten enthaltende Polymere können zu Vinylamineinheiten enthaltenden Polymerisaten hydrolysiert werden. So entstehen beispielsweise durch Abspaltung von Formylgruppen aus N-Vinylformamideinheiten enthaltenden Polymerisaten und durch Abspaltung der Gruppe CH₃-CO- aus N-Vinylacetamideinheiten enthaltenden Polymerisaten entstehen jeweils Vinylamin-Einheiten enthaltende Polymerisate. Die Abspaltung kann partiell oder vollständig durchgeführt werden. Sofern die Hydrolyse in Gegenwart von Säuren vorgenommen wird, liegen die Vinylamin-Einheiten der Polymeren als Ammoniumsalze vor. Die Hydrolyse kann jedoch auch mit Hilfe von Basen vorgenommen werden, z.B. von Metallhydroxiden, insbesondere von Alkalimetall- und Erdalkalimetallhydroxiden. Vorzugsweise verwendet man Natriumhydroxid oder Kaliumhydroxid. In besonderen Fällen kann die Hydrolyse auch mit Hilfe von Ammoniak oder Aminen durchgeführt werden. Bei der Hydrolyse in Gegenwart von Basen liegen die Vinylamin-Einheiten in Form der freien Basen vor.

Als Hydrolysemittel eignen sich vorzugsweise Mineralsäuren, wie Halogenwasserstoffe, die gasförmig oder als wässrige Lösung eingesetzt werden können. Vorzugsweise verwendet man konzentrierte Salzsäure, Schwefelsäure, Salpetersäure oder Phosphorsäure sowie organische Säuren, wie C₁- bis C₅-Carbonsäuren, sowie aliphatische oder aromatische Sulfonsäuren. Beispielsweise benötigt man pro Formylgruppenäquivalent in den N-Vinylformamideinheiten einpolymerisiert enthaltenden Polymeren 0,05 bis 2, insbesondere 1 bis 1,5 Moläquivalente einer Säure. Die Hydrolyse der N-Vinylformamid-Einheiten verläuft bedeutend schneller als die der N-Vinylacetamid-Einheiten aufweisenden Polymerisate. Sofern man Copolymerisate der in Betracht kommenden Vinylcarbonsäureamide mit anderen Comonomeren der Hydrolyse unterwirft, so können auch die im Copolymerisat enthaltenen Comonomer-Einheiten chemisch verändert werden. So entstehen beispielsweise aus Vinylacetat-Einheiten Vinylalkohol-Einheiten. Aus Acrylsäuremethylester-Einheiten entstehen bei der Hydrolyse Acrylsäure-Einheiten und aus AcrylnitrilEinheiten werden Acrylamid- bzw. Acrylsäure-Einheiten gebildet. Die Hydrolyse der N-Vinylformamid- und/oder Vinylacetamid-Einheiten der Polymerisate (A) kann zu 5 bis 100, vorzugsweise 10 bis 40 % durchgeführt werden. Obwohl die wässrigen Dispersionen von wasserlöslichen N-Vinylcarbonsäureamiden beim Verdünnen mit Wasser in Lösung gehen, wird die Dispersion bei der Hydrolyse überraschenderweise nicht zerstört. Der Teilchendurchmesser der hydrolysierten Teilchen beträgt vor bzw. nach der Hydrolyse z.B. 0,1 bis 50 µm.

Die so erhältlichen wässrigen Dispersionen von N-Vinylcarbonsäureamiden bilden beim Verdünnen mit Wasser klare wässrige Polymerlösungen. Die Viskosität der Dispersionen beträgt beispielsweise 10 bis 25 000 mPas, vorzugsweise 10 bis 15 000 mPas (bestimmt in einem Brookfield-Viskosimeter bei 20°C, Spindel Nr. 4 und 20 UpM). Die mittlere Teilchengröße der Dispersionen, die Polymerisate mit Einheiten von N-Vinylformamid, N-Vinylacetamid und/oder N-Methyl-N-vinylacetamid enthalten, haben meistens eine mittlere Teilchengröße von 0,1 bis 50 µm, vorzugsweise 0,1 bis 10 µm.

Die oben beschriebenen Dispersionen, d.h. die nicht hydrolisierten als auch die hydrolysierten wässrigen Dispersionen von wasserlöslichen N-Vinylcarbonsäureamiden werden als Entwässerungs-, Flockungs- und Retentionsmittel sowie als Nass- und Trockenfestigkeitsmittel und als Fixiermittel bei der Herstellung von Papier verwendet. Die kationischen Polymeren können außerdem als Flockungsmittel für Abwässer bei der Klärschlammentwässerung, als Flockungsmittel bei der Erzaufbereitung und der tertiären Erdölförderung oder als Dispergiermittel, z.B. für anorganische und organische Pigmente, Farbstoffe, Zement oder Pflanzenschutzmittel verwendet werden. Die nicht hydrolysierten wie auch die hydrolysierten wässrigen Dispersionen sind außerdem als Verfestigungsmittel für Papier, als Fixiermittel für lösliche und unlöslichen Störstoffe bei der Papierherstellung und als Mittel für die Papierstreicherei einsetzbar. Sie können ferner als Beschichtungsmaterial für Düngemittel und Pflanzenschutzmittel und als Fußbodenpflegemittel eingesetzt werden. Auch in der Kosmetik, z.B. als Bestandteil von haarkosmetischen Zubereitungen, wie beispielsweise Conditioner, Haarfestiger oder als Conditioner für Hautpflegemittel sowie als Verdicker für Kosmetikformulierungen ferner als Bestandteil von kosmetischen Zubereitungen für die Mundpflege sind die genannten wässrigen hydrolysierten bzw. nicht hydrolysierten Polymerdispersionen einsetzbar.

Die K-Werte wurden nach H. Fikentscher, Cellulose-Chemie, Band 13, 58-64 und 71-74 (1932) in wässriger Lösung bei 25°C und einer Konzentrationin von 0,1 % Gew.-% in 1 gew.-%iger Kochsalzlösung bestimmt. Die Viskosität der Dispersionen wurde jeweils in einem Brookfield-Viskosimeter mit einer Spindel Nr. 4 bei 20 UpM und einer Temperatur von 20°C gemessen. Die Prozentangaben in den Beispielen sind Gewichtsprozent.

### Beispiele

### Herstellung von Stabilisator 1

327,6 g eines Polyethylenglykols mit einem mittleren Molekulargewicht (Zahlenmittel) von 4000 wurden in einem 1 1 fassenden Reaktionsgefäß unter einem Stickstoffstrom auf eine Temperatur von 100°C erhitzt. Die Schmelze wurde mit einem Ankerrührer kräftig gerührt und mit der Hälfte einer Lösung von 13,20 g tert.-Butyl-peroctoat in 48,30 g Dipropylenglykol innerhalb von 15 Minuten gemischt. Die restliche Lösung des Initiators und 218,40 g N-Vinylformamid wurden gleichzeitig aber getrennt voneinander innerhalb von 1,5 Stunden bzw. einer Stunde zugegeben. Nach Zugabe des Initiators wurde die Mischung noch eine Stunde bei 100°C gerührt, mit 115 g Wasser verdünnt und auf Raumtemperatur abgekühlt. Der Feststoffgehalt der Reaktionsmischung betrug 78 %. Das so hergestellte Pfropfpolymerisat hatte einen K-Wert von 36 und einen Restmonomergehalt von 0,3 %.

### Herstellung von Stabilisator 2

327,6 g eines Polyethylenglykols mit einem mittleren Molekulargewicht (Zahlenmittel) von 4000 wurden in einen Laborkneter eingefüllt und darin in einem Stickstoffstrom unter Durchmischen auf eine Temperatur von 100°C erhitzt. Innerhalb von 15 Minuten gab man die Hälfte einer Lösung von 4,40 g tert.-Butylperoctoat in 16,10 g Dipropylenglykol zu. Die restliche Initiatorlösung und 218,40 g N-Vinylformamid wurden gleichzeitig, jedoch getrennt voneinander, innerhalb von 1,5 Stunden zugegeben. Das Reaktionsgemisch wurde anschließend noch eine Stunde bei 100°C gehalten und danach auf Raumtemperatur abgekühlt. Man erhielt ein weißes, granulatförmiges Propfpolymer mit einem K-Wert von 33.

### Beispiel 1

In einem 2 1 fassenden Reaktor aus Glas mischte man 175 g Polyethylenglykol mit einem mittleren Molekulargewicht (Zahlenmittel) von 1500, 46,3 g des Stabilisators 1, 2,5 g Natriumdihydrogenphosphat und 525 g Wasser. Der pH-Wert der Lösung wurde durch Zugabe von 50 %iger wässriger Natronlauge auf 6,7 eingestellt. 1,25 g 2,2'-Azobis(2-methylpropionamidin)dihydrochlorid und 250 g N-Vinylformamid wurden unter einer Stickstoffatmosphäre zu der Lösung gegeben. Die Reaktionsmischung wurde dann unter einem ständigen Stickstoffstrom und unter Rühren auf eine Temperatur von 50°C erhitzt und sechs Stunden bei dieser Temperatur polymerisiert. Dann fügte man 0,3 g 2,2'-Azo-bis[2-(imidazolin-2-yl)propan]dihydrochlorid zu und rührte das Reaktionsgemisch anschließend noch 1 Stunde bei einer Temperatur von 60°C. Die erhaltene milchig weiße Mischung wurde auf Raumtemperatur abgekühlt. Sie hatte eine Brookfield-Viskosität von 500 mPas (gemessen mit Spindel 4, bei 20 UpM). Der Teilchendurchmesser lag in dem Bereich von 3 - 20 µm, der Restmonomergehalt an N-Vinylformamid betrug 0,6 %. Das Polymerisat hatte einen K-Wert von 169.

### Beispiel 2

180 g der nach Beispiel 1 erhaltenen Dispersion wurden in einen 250 ml fassenden Rundkolben transferiert und darin auf eine Temperatur von 60°C erhitzt. Nachdem der Kolbeninhalt diese Temperatur erreicht hatte, gab man 15,3 g konzentrierte Schwefelsäure langsam zu, erhitzte den Kolbeninhalt 4 Stunden unter Rühren auf 60°C und kühlte ihn danach auf Raumtemperatur ab. Die resultierende milchig weiße Mischung hatte eine Brookfiled-Viskosität von 640 mPas (Spindel 4, 20 UpM). Der Durchmesser der dispergierten Teilchen betrug unverändert 3 bis 20 µm. Der Hydrolysegrad des Polyvinylformamids betrug 31% (bestimmt durch potentiometrische Titration).

### Beispiel 3

In einem 5 1 fassenden Reaktor aus Glas wurden nacheinander folgende Komponenten gemischt: 563 g Polyethylenglykol mit einem Molekulargewicht nach dem Zahlenmittel von 1500, 122 g Stabilisator 2, 8,0 g Natriumdihydrogenphosphat und 1707 g Wasser. Durch Zugabe von 50 %iger wässriger Natronlauge wurde der pH-Wert der Lösung auf 6,7 eingestellt. Unter einer Stickstoffatmosphäre gab man 1,25 g 2,2'-Azobis(2-methylpropionamidin)dihydrochlorid und 250 g N-Vinylformamid zu und erhitzte die Mischung unter ständigem Rühren unter Stickstoff auf eine Temperatur von 50°C. Sie wurde 6 Stunden bei dieser Temperatur gerührt, danach mit 0,5 g 2,2'-Azobis[2-(imidazolin-2-yl)propan]dihydrochlorid versetzt und 1,5 Stunden bei 60°C gehalten. Nach dem Abkühlen auf Raumtemperatur erhielt man eine milchig weiß aussehende Dispersion, die eine Brookfield-Viskosität (Spindel 4, 20 UpM) von 960 mPas und einen Durchmesser der dispergierten Polymerteilchen von 1 µm hatte. Das Polymerisat hatte einen K-Wert von 171 und einen Restmonomerngehalt an N-Vinylformamid von 0,3 %.

### Beispiel 4

180 g der nach Beispiel 3 erhaltenen Dispersion wurden in einen 250 ml fassenden Rundkolben transferiert und darin auf eine Temperatur von 60°C erhitzt. Nachdem der Kolbeninhalt diese Temperatur erreicht hatte, gab man langsam 15,3 g konzentrierte Schwefelsäure zu, erhitzte den Kolbeninhalt 4 Stunden unter Rühren auf 60°C und kühlte ihn danach auf Raumtemperatur ab. Die resultierende milchig weiße Mischung hatte eine Brookfiled-Viskosität von 880 mPas (Spindel 4, 20 UpM). Der Durchmesser der dispergierten Teilchen betrug unverändert 1µm. Der Hydrolysegrad des Polyvinylformamids betrug 34 % (bestimmt durch potentiometrische Titration).

### Vergleichsbeispiel 1

In einem 2 1 fassenden Reaktor aus Glas legte man 175 g Polyethylenglykol mit einem mittleren Molekulargewicht (Zahlenmittel) von 1500, 2,5 g Natriumdihydrogenphosphat und 571 g Wasser vor. Der pH-Wert der Lösung wurde durch Zugabe von 50 %iger wässriger Natronlauge auf 6,7 eingestellt. Zu dieser Lösung gab man dann unter einer Stickstoffatmosphäre 1,25 g 2,2'-Azobis(2-methyl-propionamidin)dihydrochlorid und 250 g N-Vinylformamid und erhitzte die Mischung unter ständigem Rühren unter Stickstoff auf eine Temperatur von 50°C. Das Reaktionsgemisch wurde milchig weiß und koagulierte im Verlauf von ≈1 Stunde unter Bildung einer festen, gummiartigen Masse, die nicht mehr gerührt werden konnte.

## Patentansprüche

1. Wässrige Dispersionen von wasserlöslichen Polymerisaten von N-Vinylcarbonsäureamiden, **dadurch gekennzeichnet, daß** sie als Stabilisator wasserlösliche Pfropfpolymere enthalten, die durch radikalisch initiierte Polymerisation von Monomeren oder Monomergemischen aus
- 10 bis 100 Gew.-% N-Vinylcarbonsäureamiden der Formel
CH₂=CH-NR₂-CO-R¹ (I),
in der R¹, R² = H und /oder C₁- bis C₆-Alkyl bedeuten,und
- 0 bis 90 Gew.-% anderen, mit den Monomeren der Formel I copolymerisierbaren Monomeren in Gegenwart von Polymerisaten, die mindestens 3 Einheiten eines C₂- bis C₄-Alkylenoxids enthalten und/oder von Polytetrahydrofuranen, wobei das Molekulargewich der Polyalkylenglykole im Bereich kleiner 500 000 liegt (nach Zahlenmittel)
erhältlich sind.

2. Wässrige Dispersionen nach Anspruch 1, **dadurch gekennzeichnet, daß** sie als Stabilisator hydrolysierte Pfropfpolymere enthalten, die durch Abspaltung der Gruppe -CO-R¹ aus den aufgepfropften N-Vinylcarbonsäureamideinheiten des Stabilisators unter Bildung von Vinylamineinheiten erhältlich sind.

3. Wässrige Dispersionen nach Anspruch 1 oder 2, **dadurch gekennzeichnet, daß** 100 Gew.-Teile der Dispersionen (a) 5 bis 60 Gew.-Teile eines wasserlöslichen Polymerisats eines N-Vinylcarbonsäureamids und (b) 0,1 bis 30 Gew.-Teile mindestens eines Stabilisators enthalten.

4. Wässrige Dispersionen nach einer der Ansprüche 1 oder 3, **dadurch gekennzeichnet, daß** die Polymerisate Einheiten von N-Vinylformamid, N-Vinylacetamid und/oder N-Methyl-N-vinylacetamid enthalten und eine mittlere Teilchengröße von 0,1 bis 50 µm haben.

5. Wässrige Dispersionen nach einem der Ansprüche 1 bis 4, **dadurch gekennzeichnet, daß** die Polymerisate ausgewählt sind aus der Gruppe der Homopolymerisate von N-Vinylformamid, der Copolymerisate von N-Vinylformamid mit anderen monoethylenisch ungesättigten Monomeren und der daraus durch Hydrolyse jeweils erhältlichen Vinylamineinheiten enthaltenden Polymeren.

6. Wässrige Dispersionen nach einem der Ansprüche 1 bis 5, **dadurch gekennzeichnet, daß** die Viskosität der Dispersionen 10 bis 25 000 mPas, vorzugsweise 10 bis 15 000 mPas beträgt.

7. Wässrige Dispersionen nach einem der Ansprüche 1 bis 6, **dadurch gekennzeichnet, daß** die Dispersionen als Stabilisator wasserlösliche, vernetzte Pfropfpolymerisate enthalten, die durch Polymerisation von 0,01 bis 10 Gew.-Teilen mindestens eines N-Vinylcarbonsäureamids in Gegenwart von 100 Gew.-Teilen mindestens eines Polyalkylenglykols und/oder Polytetrahydrofuranserhältlich sind.

8. Verfahren zur Herstellung von wässrigen Dispersionen von wasserlöslichen Polymerisaten von N-Vinylcarbonsäureamiden durch radikalische Polymerisation von N-Vinylcarbonsäureamiden in wässrigem Medium in Gegenwart von Stabilisatoren, **dadurch gekennzeichnet, daß** man als Stabilisator und/oder Fällungsmittel wasserlösliche Pfropfpolymere einsetzt, die durch radikalisch initiierte Polymerisation von Monomeren oder Monomergemischen aus
- 10 bis 100 Gew.-% N-Vinylcarbonsäureamiden der Formel
CH₂=CH-NR²-CO-R¹ (I),
in der R¹, R²= H und /oder C₁- bis C₆-Alkyl bedeuten,und
- 0 bis 90 Gew.-% anderen, mit den Monomeren der Formel I copolymerisierbaren Monomeren in Gegenwart von Polymerisaten, die mindestens 3 Einheiten eines C₂- bis C₄-Alkylenoxids enthalten und/oder von Polytetrahydrofuranen, wobei das Molekulargewich der Polyalkylenglykole im Bereich kleiner 500 000 liegt (nach Zahlenmittel)
erhältlich sind.

9. Verfahren nach Anspruch 8, **dadurch gekennzeichnet, daß** man die Polymerisation zusätzlich in Gegenwart von wasserlöslichen polymeren Verbindungen durchführt, die die entstehenden Poly-N-Vinylcarbonsäureamide aus der wässrigen Lösung ausfällen.

10. Verfahren nach Anspruch 8 oder 9, **dadurch gekennzeichnet, daß** man als Verbindung der Formel I N-Vinylformamid einsetzt.

11. Verfahren nach einem der Ansprüche 8 bis 10, **dadurch gekennzeichnet, daß** man N-Vinylformamid als Vinylcarbonsäureamid einsetzt.

12. Verfahren nach einem der Ansprüche 8 bis 10, **dadurch gekennzeichnet, daß** man wässrige Dispersionen von Polymerisaten des N-Vinylformamids unter teilweise oder vollständiger Abspaltung von Formylgruppen aus den Vinylformamideinheiten enthaltenden Polymerisaten in Vinylamineinheiten enthaltende wässrige Dispersionen überführt.

13. Verwendung der wässrigen Dispersionen von wasserlöslichen Polymerisaten von N-Vinylcarbonsäureamiden nach den Ansprüchen 1 bis 7 als Entwässerungs-, Flockungs- und Retentionsmittel, als Naß- und Trockenfestigkeitsmittel und als Fixiermittel bei der Herstellung von Papier, sowie als Bestandteil von haarkosmetischen Zubereitungen, als Bestandteil von kosmetischen Zubereitungen für die Mundpflege, als Conditioner für Hautpflegemittel und als Verdickungsmittel für Kosmetikformulierungen.

## Claims

1. An aqueous dispersion of a water-soluble polymer of N-vinylcarboxamides, which contains, as a stabilizer, a water-soluble graft polymer which is obtainable by free radical polymerization of monomers or monomer mixtures comprising
- from 10 to 100% by weight of N-vinylcarboxamides of the formula
CH₂=CH-NR²-CO-R¹ (I),
where R¹ and R² are H and/or C₁- to C₆-alkyl,
and
- from 0 to 90% by weight of other monomers copolymerizable with the monomers of the formula I, in the presence of polymers which comprise at least 3 units of a C₂- to C₄-alkylene oxide and/or of polytetrahydrofurans, the number average molecular weight of polyalkylene glycols being less than 500 000.

2. An aqueous dispersion as claimed in claim 1, which contains, as a stabilizer, a hydrolyzed graft polymer which is obtainable by eliminating the -CO-R¹ group from the grafted-on N-vinylcarboxamide units of the stabilizer with formation of vinylamine units.

3. An aqueous dispersion as claimed in claim 1 or 2, wherein 100 parts by weight of the dispersion contain (a) from 5 to 60 parts by weight of a water-soluble polymer of an N-vinylcarboxamide and (b) from 0.1 to 30 parts by weight of at least one stabilizer.

4. An aqueous dispersion as claimed in any of claims 1 to 3, wherein the polymer contains units of N-vinylformamide, N-vinylacetamide and/or N-methyl-N-vinylacetamide and has a mean particle size of from 0.1 to 50 µm.

5. An aqueous dispersion as claimed in any of claims 1 to 4, wherein the polymer is selected from the group consisting of the homopolymers of N-vinylformamide, the copolymers of N-vinylformamide with other monoethylenically unsaturated monomers and the polymers containing vinylamine units and obtainable in each case therefrom by hydrolysis.

6. An aqueous dispersion as claimed in any of claims 1 to 5, wherein the viscosity of the dispersion is from 10 to 25 000, preferably from 10 to 15 000, mPa.s.

7. An aqueous dispersion as claimed in any of claims 1 to 6, which contains, as a stabilizer, a water-soluble, crosslinked graft polymer which is obtainable by polymerization of from 0.01 to 10 parts by weight of at least one N-vinylcarboxamide in the presence of 100 parts by weight of at least one polyalkylene glycol and/or polytetrahydrofuran.

8. A process for the preparation of an aqueous dispersion of a water-soluble polymer of N-vinylcarboxamides by free radical polymerization of N-vinylcarboxamides in an aqueous medium in the presence of a stabilizer, wherein water-soluble graft polymers which are obtainable by free radical polymerization of monomers or monomer mixtures comprising
- from 10 to 100% by weight of N-vinylcarboxamides of the formula
CH₂=CH-NR²-CO-R¹ (I),
where R¹ and R² are H and/or C₁- to C₆-alkyl,
and
- from 0 to 90% by weight of other monomers copolymerizable with the monomers of the formula I, in the presence of polymers which comprise at least 3 units of a C₂- to C₄-alkylene oxide and/or of polytetrahydrofurans, the number average molecular weight of polyalkylene glycols being less than 500 000.

9. A process as claimed in claim 8, wherein the polymerization is additionally carried out in the presence of water-soluble polymeric compounds which precipitate the resulting poly-N-vinylcarboxamides from the aqueous solution.

10. A process as claimed in claim 8 or 9, wherein N-vinylformamide is used as a compound of the formula I.

11. A process as claimed in any of claims 8 to 10, wherein the vinylcarboxamide used is N-vinylformamide.

12. A process as claimed in any of claims 8 to 10, wherein an aqueous dispersion of a polymer of N-vinylformamide is converted into an aqueous dispersion containing vinylamine units by eliminating some or all of the formyl groups from the polymer containing vinylformamide units.

13. The use of an aqueous dispersion of a water-soluble polymer of N-vinylcarboxamides as claimed in any of claims 1 to 7 as a drainage aid, flocculant or retention aid, as a wet or dry strength agent or as fixing agent in papermaking, or as a component of cosmetic hair preparations, as a component of cosmetic preparations for oral hygiene, as a conditioner for hair setting compositions and as a thickener for cosmetic formulations.

## Revendications

1. Dispersions aqueuses de polymères hydrosolubles d'amides d'acides N-vinylcarboxyliques, **caractérisées en ce qu'**elles contiennent, comme stabilisateur, des polymères greffés hydrosolubles, que l'on obtient par polymérisation initiée par voie radicalaire de monomères ou de mélanges de monomères constitués de :
- 10 à 100 % en poids d'amides d'acides N-vinylcarboxyliques de formule :
CH₂=CH-NR²-CO-R¹ (I)
dans laquelle R¹, R² représentent de l'hydrogène et/ou un alkyle en C₁-C₆,
- 0 à 90 % en poids d'autres monomères copolymérisables avec les monomères de formule I en présence de polymères qui contiennent au moins 3 unités d'un oxyde d'alkylène en C₂-C₄ et/ou de polytétrahydrofurannes, dans lesquels le poids moléculaire des polyalkylèneglycols se situe dans la plage de valeurs inférieures à 500 000 (en moyenne arithmétique).

2. Dispersions aqueuses selon la revendication 1, **caractérisées en ce qu'**elles contiennent, comme stabilisateur, des polymères greffés hydrolysés, que l'on peut obtenir en éliminant le groupe -CO-R¹ par clivage des unités greffées d'amides d'acides N-vinylcarboxyliques du stabilisateur, tout en permettant la formation d'unités vinylamine.

3. Dispersions aqueuses selon la revendication 1 ou 2, **caractérisées en ce que** 100 parties en poids des dispersions contiennent (a) 5 à 60 parties en poids d'un polymère hydrosoluble d'un amide d'acide N-vinylcarboxylique et (b) 0,1 à 30 parties en poids d'au moins un stabilisateur.

4. Dispersions aqueuses selon la revendication 1 ou 3, **caractérisées en ce que** les polymères contiennent des unités N-vinylformamide, N-vinylacétamide et/ou N-méthyl-N-vinylacétamide, et présentent une taille de particule moyenne de 0,1 à 50 µm.

5. Dispersions aqueuses selon l'une quelconque des revendications 1 à 4, **caractérisées en ce que** les polymères sont choisis dans le groupe des homopolymères de N-vinylformamide, des copolymères de N-vinylformamide avec d'autres monomères à insaturation monoéthylénique et des polymères contenant des unités vinylamine que l'on peut obtenir, respectivement, par hydrolyse de ceux-ci.

6. Dispersions aqueuses selon l'une quelconque des revendications 1 à 5, **caractérisées en ce que** la viscosité des dispersions se situe dans la plage de 10 à 25 000 mPas, de préférence de 10 à 15 000 mPas.

7. Dispersions aqueuses selon l'une quelconque des revendications 1 à 6, **caractérisées en ce que** les dispersions contiennent, comme stabilisateur, des polymères greffés réticulés hydrosolubles, que l'on peut obtenir par polymérisation de 0,01 à 10 parties en poids d'au moins un amide d'acide N-vinylcarboxylique en présence de 100 parties en poids d'au moins un polyalkylèneglycol et/ou d'un polytétrahydrofuranne.

8. Procédé de préparation de dispersions aqueuses de polymères hydrosolubles d'amides d'acides N-vinylcarboxyliques par polymérisation par voie radicalaire d'amides d'acides N-vinylcarboxyliques en milieu aqueux en présence de stabilisateurs, **caractérisé en ce que** l'on utilise comme stabilisateur et/ou agent de précipitation des polymères greffés hydrosolubles, que l'on peut obtenir par polymérisation initiée par voie radicalaire de monomères ou de mélanges de monomères constitués de :
- 10 à 100 % en poids d'amides d'acides N-vinylcarboxyliques de formule:
CH₂=CH-NR²-CO-R¹ (I)
dans laquelle R¹, R² représentent de l'hydrogène et/ou un alkyle en C₁-C₆,
- 0 à 90 % en poids d'autres monomères copolymérisables avec les monomères de formule I en présence de polymères qui contiennent au moins 3 unités d'un oxyde d'alkylène en C₂-C₄ et/ou de polytétrahydrofurannes, le poids moléculaire des polyalkylèneglycols se situant dans la plage de valeurs inférieures à 500 000 (en moyenne arithmétique).

9. Procédé selon la revendication 8, **caractérisé en ce que** l'on effectue la polymérisation également en présence de composés polymères hydrosolubles, qui entraînent la précipitation des poly(amides d'acides N-vinylcarboxyliques) formés à partir de la solution aqueuse.

10. Procédé selon la revendication 8 ou 9, **caractérisé en ce que** l'on utilise le N-vinylformamide comme composé de formule I.

11. Procédé selon l'une quelconque des revendications 8 à 10, **caractérisé en ce que** l'on utilise le N-vinylformamide comme amide d'acide vinylcarboxylique.

12. Procédé selon l'une quelconque des revendications 8 à 10, **caractérisé en ce que** l'on transforme des dispersions aqueuses de polymères du N-vinylformamide, moyennant le clivage partiel ou total des groupes formyle des polymères contenant des unités vinylformamide, en dispersions aqueuses contenant des unités vinylamine.

13. Utilisation des dispersions aqueuses de polymères hydrosolubles d'amides d'acides N-vinylcarboxyliques selon les revendications 1 à 7, comme agents de déshydratation, de floculation et de rétention, comme agent de résistance à l'état humide et de résistance à l'état sec et comme agent de fixation dans la fabrication du papier, ainsi que comme composant de préparations cosmétiques pour les cheveux, comme composant de préparations cosmétiques de soins de la bouche, comme conditionneurs pour des produits de soins de la peau et comme épaississant pour des formulations cosmétiques.
